# EUROPEAN PATENT SPECIFICATION

(11) **EP 2 150 186 B1**
(45) Date of publication and mention of the grant of the patent: **11.11.2015**
(21) Application number: 08745070.6
(22) Date of filing: 04.04.2008
(51) Int. Cl.: A61B 17/34, A61B 17/32

(54) **VISUALIZED ENTRY TROCAR WITH MOVING BLADE**
VISUALISIERTER EINTRITTSTROKAR MIT BEWEGLICHER KLINGE
TROCART D'OBSERVATION ET D'INTERVENTION À LAME MOBILE

(30) Priority: 11.04.2007 US 922841 P
(43) Date of publication of application: 10.02.2010
(73) Proprietor: Covidien LP, Mansfield, MA 02048 (US)
(72) Inventor: OKONIEWSKI, Gregory, G., North Haven, CT 06473 (US)
(74) Representative: Soames, Candida Jane
(86) International application number: PCT/US2008/059342
(87) International publication number: WO 2008/127887

(56) References cited:
- US-A- 5 591 186
- US-A- 5 860 996
- US-A1- 2005 273 116

## Description

### BACKGROUND

### Technical Field

The present disclosure relates to an apparatus for penetrating and for observing penetration of body tissue and, more particularly, relates to an optical trocar assembly having a reciprocating and laterally translating cutting blade, which facilitates penetration of the peritoneum or other body tissue under direct observation.

### Background of Related Art

Endoscopic surgical procedures, that is, surgical procedures performed through tubular sleeves or cannulas, have been utilized for many years. Initially, endoscopic surgical procedures were primarily diagnostic in nature. More recently as endoscopic technology has advanced, surgeons are performing increasingly complex and innovative endoscopic surgical procedures. In endoscopic procedures, surgery is performed in any hollow viscus of the body through a small incision or through narrow endoscopic tubes (cannulas) inserted through small entrance wounds in the skin. In laparoscopic procedures surgery is performed in the interior of the abdomen.

Laparoscopic procedures generally utilize instrumentation that is internally sealed to inhibit gases from entering or exiting the body through the laparoscopic or endoscopic incision. This is particularly true in surgical procedures in which the surgical region is insufflated. Moreover, laparoscopic and endoscopic procedures often require the surgeon to act on organs, tissues and vessels far removed from the incision, thereby requiring that any instruments to be used in such procedures be of sufficient size and length to permit remote operation. Typically, after the surgical region is insufflated, trocars are used to puncture the body cavity and include a cannula which remains in place for use during endoscopic procedures. Generally, trocars used during such procedures include a stylet or obturator having a sharp tip for penetrating the body cavity positioned coaxially within protective tubes to protect a patient or surgeon from inadvertent contact with the tip. An example of a known trocar is described in commonly assigned, U.S. Pat. No. 5,860,996 to Urban, et al. , which discloses the features of the preamble of claim 1.

It would be advantageous to provide a trocar assembly for observing the penetration of the peritoneum or other body portions. The trocar assembly described herein includes an obturator incorporating a trigger mechanism for selectively reciprocating a cutting blade and a translation mechanism for selectively translating the cutting blade, each of which facilitates penetration of body tissue.

### SUMMARY

The present invention is defined in the appended claims.

Accordingly, an obturator for penetrating tissue includes an outer member defining a longitudinal axis, and having proximal and distal ends, a leading member disposed adjacent the distal end of the outer member and having a tissue contacting outer surface, and a blade mounted adjacent to the leading member. The blade is adapted for at least traversing movement relative to the longitudinal axis to thereby traverse the outer surface of the leading member to facilitate penetrating of tissue during advancement of the leading member within the tissue. The leading member may include an optical window adapted to permit passage of light therethrough for detection by a clinician.

The outer member may further include a longitudinal opening adapted for reception of an endoscope. Alternatively, the obturator may include an imaging device associated with the outer member. The imaging device is adapted to transmit an image received through the optical window. In one embodiment, the blade is adapted for reciprocating movement along the outer surface of the leading member. The outer surface of the leading member may include a channel for at least partially receiving the blade. The blade may be movable within the channel. The outer member may further include an outer groove in general alignment with the channel of the leading member for at least partially receiving the blade. The blade may be movable within the outer groove. The outer member may also include a pair of opposed outer grooves. As a further alternative, the blade is adapted for longitudinal movement relative to the leading member to move between an initial position and an advanced position.

The obturator may further include a housing connected to the proximal end of the outer member. A manual actuator is mounted to the housing and operatively connected to the blade. The manual actuator may be dimensioned for manipulation by the clinician to cause at least traversing movement of the blade. In another embodiment, a manual advancer is mounted to the housing and operatively connected to the blade. The manual advancer is dimensioned for manipulation by the clinician to move the blade in a longitudinal direction between the initial position and the advanced position.

In an alternate embodiment, an optical obturator includes an outer sleeve member defining a longitudinal axis and having a longitudinal opening for reception of an endoscope, an optical member disposed adjacent the distal end of the outer sleeve member and having a tissue contacting outer surface, and being adapted to transfer an image of an object for detection by the endoscope, and a blade mounted adjacent the optical member. The blade is adapted for at least lateral traversing movement relative to the longitudinal axis to thereby traverse the outer surface of the leading member to facilitate penetrating of tissue during visualized advancement of the optical member within the tissue. The optical member may define a general hemispherically-shaped configuration. The tissue contacting surface may include an outer channel for at least partial reception of the blade. The blade is adapted to slide within the channel during the lateral traversing movement thereof. The blade further may be adapted for longitudinal movement between an initial position and an advanced position wherein the blade at least partially extends beyond the optical member. A manual advancer may be operatively connected to the blade to cause corresponding movement of the blade between the initial position and the advanced position.

### BRIEF DESCRIPTION OF THE DRAWINGS

The foregoing summary, as well as the following detailed description will be better understood when read in conjunction with the appended figures. For the purpose of illustrating the present disclosure, a preferred embodiment is shown. It is understood, however, that the present disclosure is not limited to the precise arrangement and instrumentalities shown.
FIG. 1 is a perspective view of the optical trocar, illustrating a cannula assembly, an obturator assembly, and an endoscope;
FIG. 2 is an exploded view of the handle of the obturator assembly illustrating a trigger assembly for deploying a blade and a translation mechanism for laterally translating the blade;
FIG. 3 is a cross-sectional view of the distal end of an alternate obturator assembly;
FIG. 4 is a side view in partial cross-section of the obturator assembly and the endoscope, illustrating the trigger assembly;
FIG. 5A is an enlarged side view in partial cross-section of the trigger assembly illustrating the trigger in a non-actuated position and the translation assembly in a second or laterally translated position;
FIG. 5B is an enlarged side view illustrating the mechanism for laterally translating the cutting blade, in a first or initial position.
FIG. 6 is an enlarged assembled view of the distal end of the obturator assembly illustrating the interconnection between blade pusher arms and the blade;
FIG. 7 is a sectional assembled view of a portion of the trigger assembly of FIG. 2, illustrating the cutting blade;
FIG. 8 is a side view in partial cross-section of the obturator assembly and endoscope illustrating partial actuation of the trigger assembly with the blade in the non-deployed position and the translation assembly is in a first or initial condition;
FIG. 9A is an enlarged side view of the trigger assembly of FIG. 8;
FIG. 9B is an enlarged side view of the mechanism for laterally translating the cutting blade of the apparatus of FIGS. 8 and 9A, in a second or laterally translated condition.
FIG. 10 is an enlarged view of the distal end of the obturator assembly, illustrating a dome-shaped objective optical member with the blade in the non-deployed position;
FIG. 11 is a side view in partial cross-section of the obturator assembly and endoscope illustrating actuation of the trigger assembly and the blade in the deployed position;
FIG. 12 is an enlarged side view of the trigger assembly;
FIG. 13 is an enlarged view of the distal end of the obturator assembly, illustrating the dome-shaped objective optical member and the blade in the deployed position;
FIG. 14A is an enlarged side view illustrating an alternate embodiment of a mechanism for laterally translating the cutting blade, in a first or initial position; and
FIG. 14B is an enlarged side view of the mechanism for laterally translating the cutting blade of the FIG. 14, in a second or laterally translated condition.

### DETAILED DESCRIPTION OF PREFERRED EMBODIMENTS

Embodiments of the presently disclosed optical trocar assembly will now be described in detail with reference to the drawings, in which like reference numerals designate identical or corresponding elements in each of the several views. As used herein, the term "distal" refers to that portion of the instrument, or component thereof which is further from the user while the term "proximal" refers to that point of the instrument or component thereof which is closer to the user.

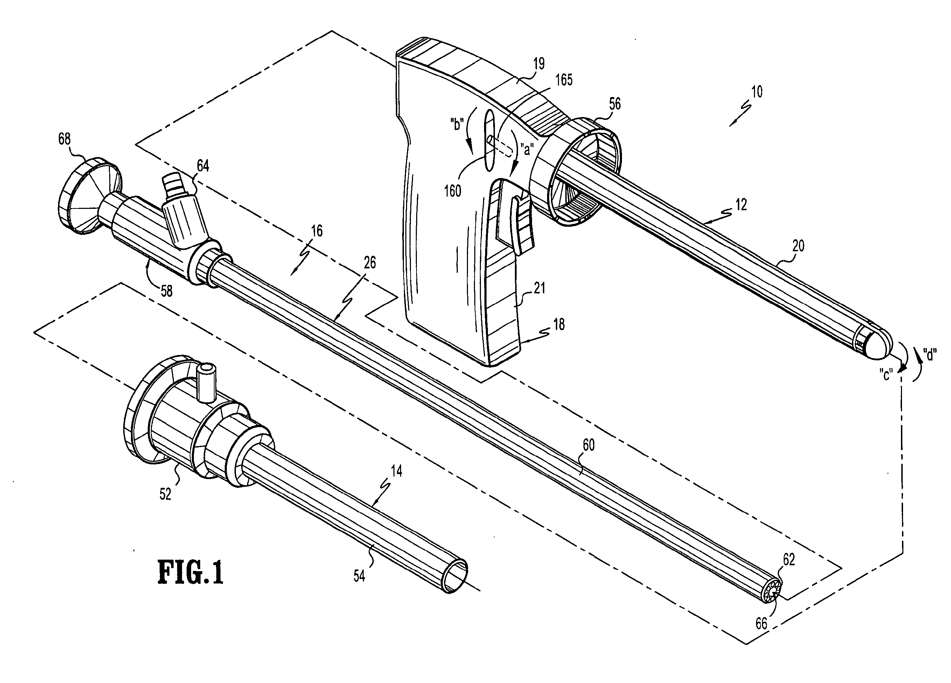

An apparatus is provided to penetrate body tissue, e.g., the abdominal wall, and to provide a simultaneous forward directional view of the body tissue being penetrated. In a preferred embodiment, shown in FIG. 1, the apparatus includes a trocar assembly 10 having an obturator assembly 12, a cannula assembly 14, and an imaging member, such as an endoscope 16. Endoscope 16 is positioned within the obturator assembly 12 to provide observation of the body tissue being penetrated. The term obturator assembly as used herein refers to the tissue penetrating assembly of the trocar assembly 10.

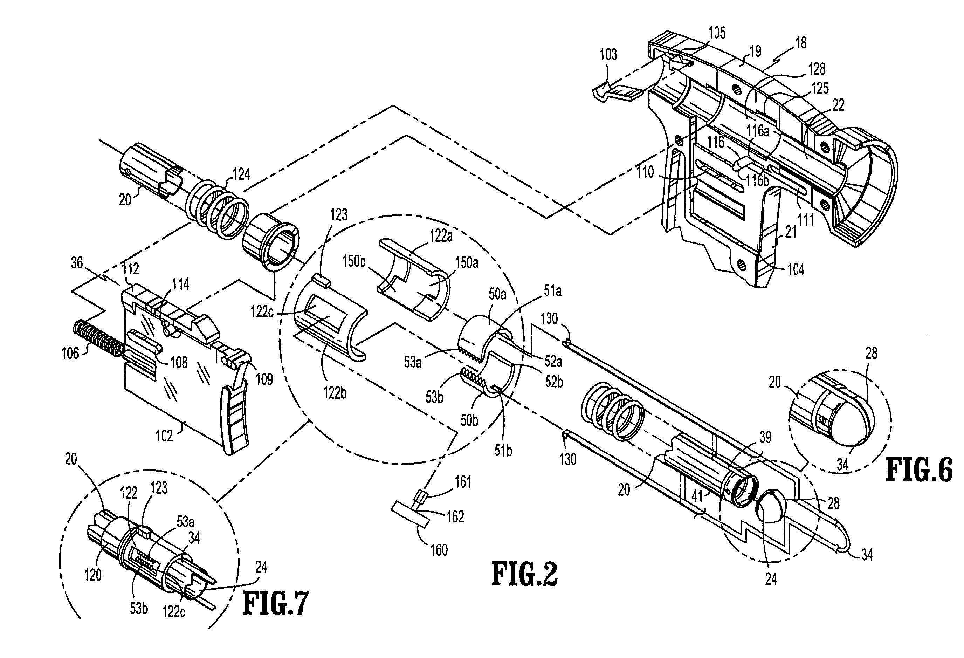

Referring to FIGS. 1 and 2, obturator assembly 12 includes housing 18 and a longitudinally extending obturator sleeve 20. Obturator housing 18 includes barrel portion 19 and hand grip 21. Rotatably mounted to obturator housing 18 is a blade translation lever 160. The proximal end of obturator sleeve 20 is secured within channel 22 of barrel portion 19 so that the obturator sleeve 20 extends outwardly from the obturator housing 18. Hand grip 21 is provided for manual gripping by the clinician to facilitate manipulation about the operative site.

Obturator sleeve 20 has a longitudinal bore 24 which extends between the proximal end and the distal end of the obturator sleeve 20. The longitudinal bore 24 is configured and dimensioned to receive the endoscopic portion 26 of the endoscope 16, as shown in FIG. 1. Housing 18 of obturator assembly 12 is constructed of two half-sections which are joined together by welding, adhesives or the like. A leaf spring 103 is positioned within channel 105 at the proximal end of the barrel portion 19 of housing 18, as shown in FIG. 2. Leaf spring 103 is provided to engage endoscopic portion 26 of endoscope 16, to frictionally maintain the endoscope in a fixed longitudinal relationship with respect to obturator sleeve 20.

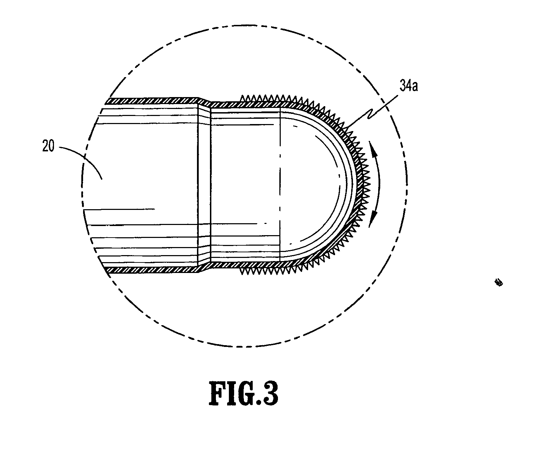

Referring to FIGS. 2 and 3, an image passing member 28 is secured to the distal end of obturator sleeve 20 and is provided to permit or direct images into the obturator sleeve 20 and/or to allow for the passage of illumination light from the obturator sleeve 20 to body tissue. The image passing member 28 may be a transparent optical window or an optical lens fabricated from a variety of materials such as polystyrene, polymethylmethacrylate (PMMA), polyurethane, transparent epoxies and/or glass or other transparent materials. The optical window shown in this preferred embodiment is hemispherical shaped, i.e., dome-shaped, and capable of allowing optical images to pass therethrough and into the longitudinal bore 24 of obturator sleeve 20, so as to impinge the distal end of endoscope 16.

The objective optical member is also a dome-shaped member. However in this configuration, optical images which impinge the dome-shaped surface of objective optical member 28 are directed into longitudinal bore 24 of obturator sleeve 20, so as to impinge the distal end of endoscope 16. The objective optical member as well as the optical window is preferably configured to allow approximately a full forward angle of view.

Referring again to FIG. 2, obturator assembly 12 includes a cutting blade 34 operably connected to actuating assembly 36. Cutting blade 34 is preferably a wire, band or the like, capable of conforming to the outer surface of the dome-shaped image passing member 28. Cutting blade 34, or at least a portion thereof, may be sharpened or serrated 34a (FIG. 3) for assisting in penetration of tissue. The sharpened or serrated portion of cutting blade 34 may be varied to correspond to the amount of lateral translation experienced by cutting blade 34. Cutting blade 34 interfits within an arcuate recess in the dome-shaped image passing member 28 when in a non-deployed position. Cutting blade 34 is preferably centered with respect to the outer surface of the image passing member as shown. Thus, in visualization, the cutting blade is seen as a thin line through the center, i.e. bisecting, the viewing field so as not to obstruct viewing of the body.

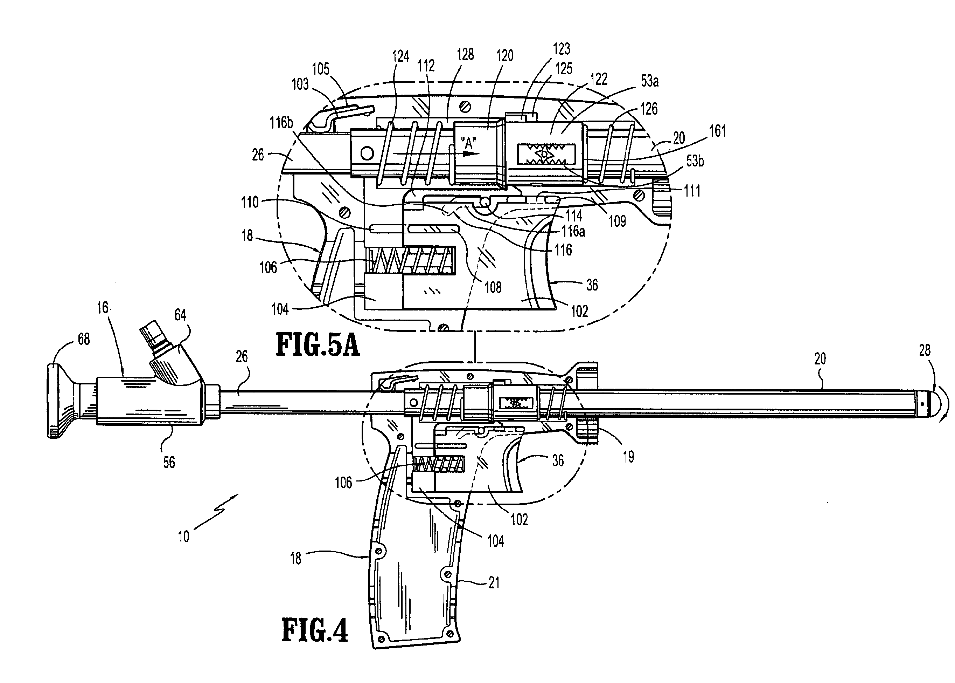

Referring now to FIGS. 2, 4, 5 and 6, actuating assembly 36 is contained within housing 18 and is provided to move cutting blade 34 in general longitudinal directions between a non-deployed position (FIG. 4) and a deployed position (FIG. 6) which will be described in more detail below. As shown in FIG. 2, the actuating assembly 36 includes a trigger 102 slidably positioned within channel 104 in housing 18 and movable between non-actuating and actuating positions. Spring 106 is secured between housing 18 and trigger 102 so as to normally bias the trigger to the non-actuating position, shown in FIG. 5A. Alignment fingers 108 and 109 extend from trigger 102 into corresponding channels 110 and 111 within housing 18. Alignment fingers 108 and 109 are provided to maintain the alignment of trigger 102 within channel 104 of housing 18.

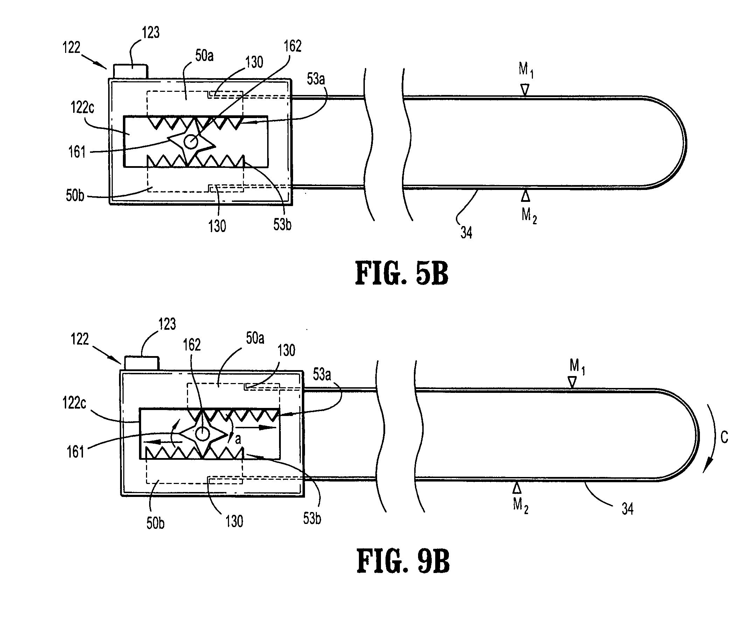

Hammer blade drive latch 112 is secured to or extends integrally from trigger 102 and includes a latch release member, in the form of post 114. Post 114 extends between the two housing halves and into corresponding channels 116 of each housing half, as shown in FIG. 5. Channels 116 include a longitudinal portion 116*a* which permits the hammer latch 112 to engage the hammer, and a sloped portion 116*b* which causes hammer latch 112 to disengage from the hammer, as will be described in more detail below.

Referring again to FIGS. 2 and 5, the actuating assembly 36 also includes blade drive members, such as hammer 120, bushing assembly 122 and a pair of drive springs 124 and 126. As shown in FIG. 5, the hammer, bushing and drive springs are coaxially aligned with obturator sleeve 20. Drive spring 124 is positioned about obturator sleeve 20 within channel 128 of each housing half so that one end of the spring engages the housing and the other end engages the proximal end of hammer 120. Drive spring 124 normally biases hammer 120 toward the distal end of the obturator assembly 12, indicated by arrow "A" in FIG. 5. The proximal end of bushing assembly 122 is positioned adjacent hammer 120 and the distal end of bushing assembly 122 engages one end of drive spring 126. The other end of drive spring 126 engages the housing 18, as shown. Finger 123 extending from bushing assembly 122 into channel 125 within housing 18, are provided to limit the proximal and distal movement of the bushing assembly 122 and thus the proximal and distal movement of blade 34.

Referring to FIGS. 2 and 7, cutting blade 34 is positioned in slots 39 and 41 of obturator sleeve 20. The proximal ends of cutting blade 34 include fingers 130 extending outwardly therefrom which are configured to slide within and operatively connect to bushing assembly 122. Cutting blade 34 may be configured for releasable engagement with bushing assembly 122. Preferably cutting blade 34 may be replaced between procedures or as the sharpened or serrated portion becomes dull.

In addition to being configured for proximal and distal advancement of cutting blade 34, bushing assembly 122 is further configured to permit lateral translation of cutting blade 34 about the distal end of obturator 20. Bushing assembly 122 comprises first and second housing halves 122a, 122b. First and second housing halves 122a, 122b are substantially mirror images, each forming recesses 150a, 150b. Recesses 150a, 150b are configured to slideably maintain a top and a bottom blade mount 50a, 50b positioned therein. Second housing half 122b includes an opening 122c configured to enable access to top and bottom blade mounts 50a, 50b.

Blade mounts 50a, 50b are mirror image, semi-arcuate members having first and second opposing ends 52a, 52b, 53a, 53b, respectively. First ends 52a, 52b are configured to slideably engage one another when blade mounts 50a, 50b are assembled about obturator 20. First ends 52a, 52b may include a tongue and groove configuration, or some other suitable method for permitting top blade mount 50a to slide longitudinally relative to bottom blade mount 50b. First and second housing halves 122a, 122b may further be configured to retain top and bottom blade mounts 50a, 50b. Second ends 53a, 53b form toothed or geared portions that remain in a spaced apart relationship when assembled about obturator 20 (FIG. 7). Blade mounts 50a, 50b include slots 51a, 51b, respectively, for receiving proximal ends 130 of cutting blade 34.

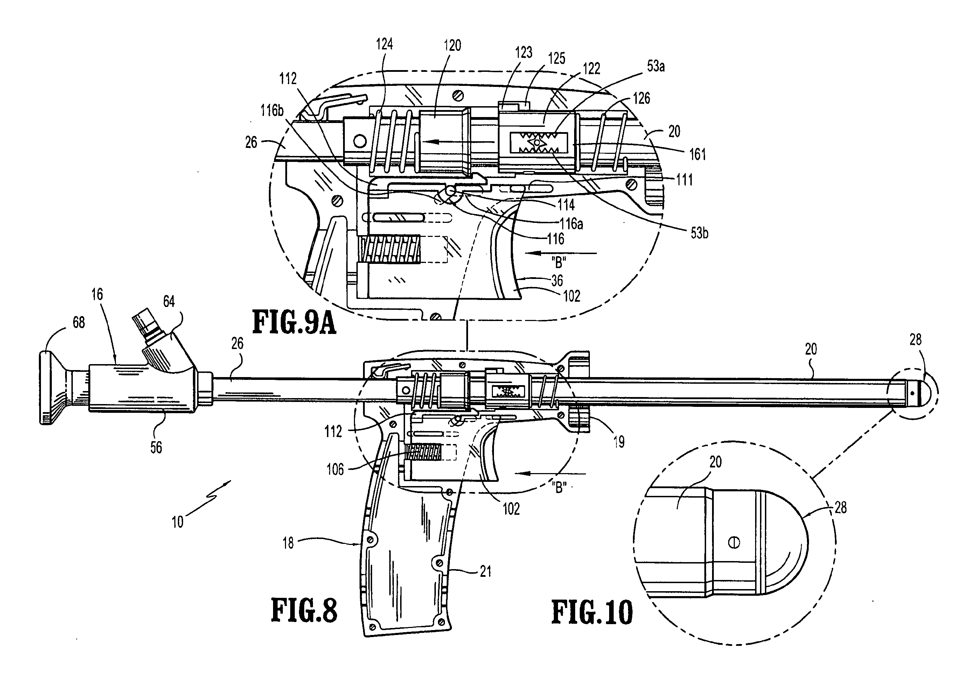

Still referring to FIGS. 2 and 7, in an assembled configuration, blade mounts 50a, 50b form an annular sleeve that is maintained about obturator 20 and within recesses 150a, 150b of bushing assembly 122. Slots 51a, 51b receive proximal ends 130 of wire cutting blade 34. Initially blade mounts 50a, 50b are positioned within recesses 150a, 150b such that they are aligned with one another (FIGS. 7 and 9). In this manner, proximal ends 130 of wire cutting blade 34 are aligned and an equal length of blade 34 is positioned above and below obturator 20. A toothed gear 61 mounted on a shaft 62 extends through opening 165 formed in obturator housing 18 and opening 122c formed in bushing assembly 122 to operably engage toothed ends 53a, 53b of top and bottom blade mounts 50a, 50b, respectively. Toothed gear 161 is operably connected to blade translation lever or knob 160 (FIG. 1) which is rotatably mounted on the exterior of obturator housing 18.

With reference now to FIGS. 1 and 5A-5B, rotation of lever 160 in a first or clockwise direction (arrow "a") causes toothed gear 161 to rotate in the same first direction. Rotation of toothed gear 161 causes engagement with and movement of toothed ends 53a, 53b of top and bottom blade mount 50a, 50b, respectively. As toothed gear 161 rotates in the first direction top blade mount 50a is advanced distally within recess 150a along obturator 20, while bottom blade mount 50a is advanced in the opposite or proximal direction within recess 150b along obturator 20. As blade mounts 50a, 50b advance opposite one another, cutting blade 34, operably connected thereto, is laterally translated (arrow "c") about the distal end of obturator 20. This lateral translation of cutting blade 34 about the distal end of obturator 20 creates a cutting motion for encouraging penetration of tissue.

Recesses 150a, 150b formed in bushing assembly 122 are configured to prevent blade mounts 50a, 50b from advancing beyond a predetermined range. Bushing assembly 122 and blade mounts 50a, 50b may be configured to permit a greater or lesser amount of lateral translation of cutting blade 34. In an alternate embodiment, bushing assembly 122 may be configured to permit advancement of top and bottom blade mounts 50a, 50b in either a proximal or distal direction from the initial position. Upon complete advancement of blade mount 50a, 50b within recesses 150a, 150b, lever 160 is prevented from further rotation in the first or clockwise direction. Lever 160 may include an indicator or be configured to show the relative position of blade mounts 50a, 50b with bushing assembly 122. Rotation of lever 160 in a second or counter-clockwise direction (arrow "b") causes blade mounts 50a, 50b to return to their initial, aligned positions (FIG. 7 and 9A-9B). In this manner, cutting blade 34 is again laterally translated about the distal end of obturator 20 to create a cutting motion, this time however, in the opposite direction (arrow "d").

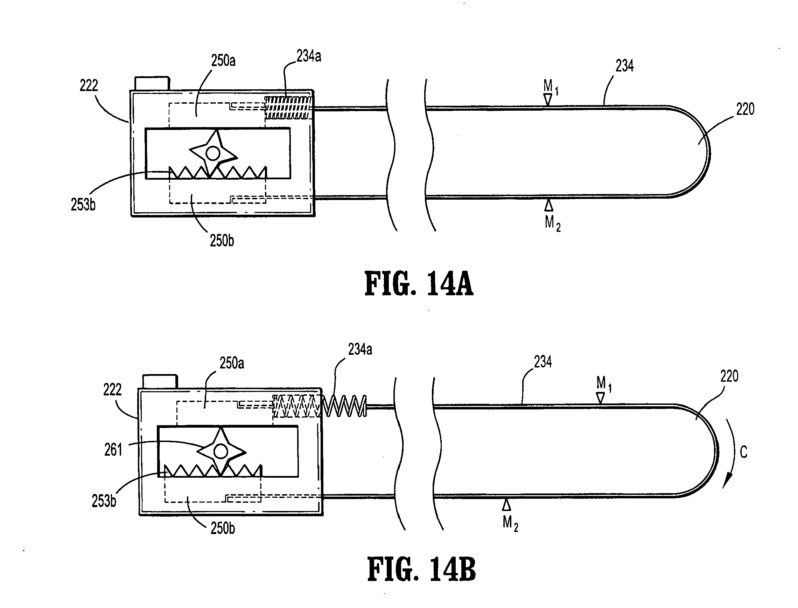

With reference now to FIGS. 14A and 14B, in an alternate embodiment of the mechanism for providing lateral translation of blade 234 about the distal end of obturator 20 first blade mount 250a may be rigidly affixed within busing assembly 222 while second blade mount 250b is permitted to laterally translate in the manner described above. Thus, as toothed gear 261 rotates and engages toothed end 253b of second blade mount 250b, second blade mount 250b is laterally translate while first blade mount 250a remains stationary (FIG. 14B). Blade 234 is configured to include a spring portion 234a for permitting lateral translation of blade 234 in a first direction about the distal end of obturator 20. Once the force turning toothed gear 261 is removed, spring portion 234a of cutting blade 234 cause the lateral retraction of blade 334. Thus, second blade mount 250b is returned to an initial position (FIG. 14A).

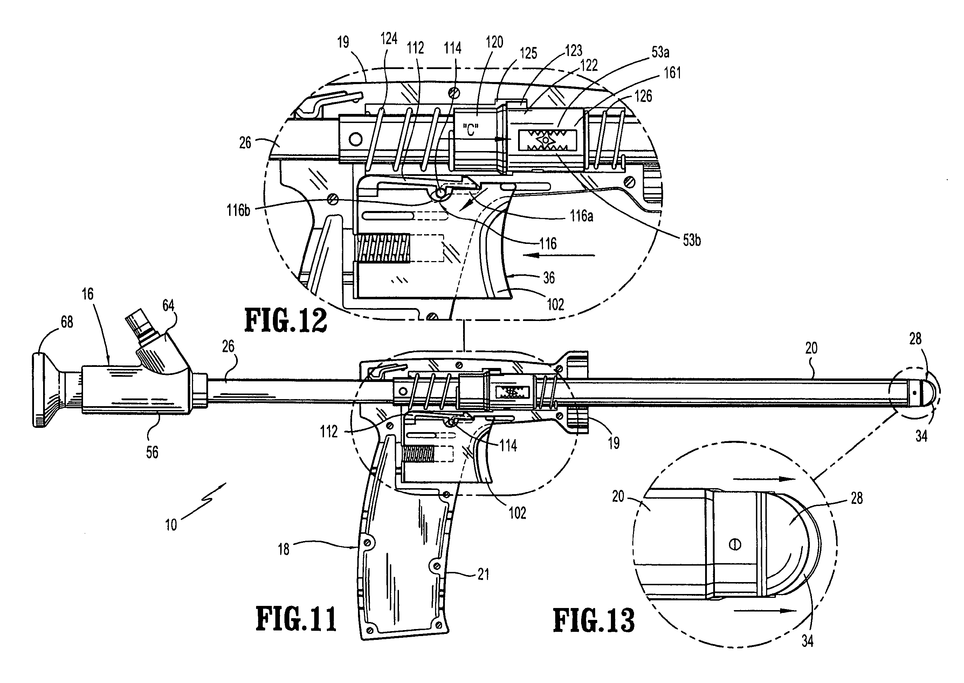

Referring back to FIGS. 8-13, in the above configuration, movement of trigger 102 in the proximal direction, shown by arrow "B" in FIGS. 8 and 9A, causes hammer latch 112 to retract hammer 120 and compress drive spring 124 (i.e., the hammer latch moves the hammer to a cocked or armed position). Post 114 is within the longitudinal portion 116a of channel 116 and blade 34 continues to remain in the non-deployed (i.e., retracted) position within objective optical member 28, as shown in FIG. 10. Further proximal movement of trigger 102 causes post 114 to move in a downward direction within the sloped portion 116b of channel 116, as shown in FIGS. 11 and 12. Downward movement of post 114 causes hammer latch 112 to disengage from hammer 120 so that hammer 120 is thrusted distally (i.e., in the direction of arrow "C") by drive spring 124. As hammer 120 moves distally, the hammer engages bushing assembly 122 and thrusts the bushing distally so as to longitudinally move cutting blade 34 to the advanced deployed (i.e., exposed) position, as shown in FIGS. 11 and 13. Distal movement of bushing assembly 122 also compresses drive spring 126 and when the biasing force of drive spring 126 exceeds the compression force exerted by the hammer 120, drive spring 126 automatically biases bushing 124 proximally so that blade 34 is automatically returned to the non-deployed position. Thus, engagement of hammer 120 and bushing assembly 122 provides substantially instantaneous deployment and retraction of the blade so the blade remains exposed for a short period of time. Thus, once the trigger is pulled to a predetermined position, blade 34 is deployed and then retracted without further action of the user (i.e., without further movement of the trigger).

Housing 18 includes opening 165 to permit lever 160 and shaft 62 extending therethrough, to move relative to bushing assembly 122 during deployment and retraction of blade 34. Thus, as cutting blade 34 is deployed and retracted blade mounts 50a, 50b move relative to and along with bushing assembly 122. In an alternate embodiment, housing 18, and more particularly opening 165, and shaft 162 and/or lever 60 may be configured such that as bushing assembly 122 is deployed or retracted, shaft 62 and thus, toothed gear 61 is rotated. As described above, rotation of toothed gear 161 causes the advancement of blade mounts 50a, 50b, thereby causing the lateral translation of cutting blade 34. In this manner, cutting blade 34 may both longitudinally reciprocate back and forth and translate laterally about the distal end of obturator 20. Lever 160 and shaft 162 may further be configured for selective engagement with housing 18, thereby permitting selective translation of cutting blade 34 about the distal end of obturator 20 when in either the deployed or retracted position.

In the configuration described, the actuation assembly 36 operates in a two step manner. In the first step, trigger 102 is moved proximally to cock hammer 120. In the second step, further proximal movement of trigger 102 causes the hammer 120 to automatically move distally to advance the blade 34 to the deployed position, and the blade is automatically returned to the non-deployed position under the force of drive spring 126. This two step manner automatically occurs upon fully squeezing trigger 102.

Referring again to FIG. 1, cannula assembly 14 includes cannula housing 52 and cannula sleeve 54 secured to the cannula housing 52 and extending outwardly therefrom. Barrel portion 19 of obturator housing 18 includes bushing 56 which is configured and dimensioned to interfit with the proximal end of cannula housing 52, so that obturator sleeve 20 coaxially aligns with cannula sleeve 54 when the two assemblies are interfitted. The cannula sleeve 54 is adapted to remain in the body after penetration and subsequent removal of the obturator assembly 12 (and endoscope 16) to allow insertion of appropriate endoscopic/laparoscopic instrumentation therethrough.

To maintain a gas tight seal within the cannula housing, a sealing member or system may be positioned therewithin which is adapted to receive the obturator assembly 12 of the present invention as well as other endoscopic surgical instruments. One example of a suitable sealing system utilizes a duckbill sealing member. A more detailed description of an exemplary cannula assembly and sealing system is found in U.S. Pat. No. 5,180,373 issued Jan. 19, 1993.

Continuing to refer to FIG. 1, endoscope 16 includes endoscopic portion 26 and endoscope housing 58. Endoscopic portion 26 is configured to transfer illuminating light from endoscope housing 58 to the distal end of the endoscopic portion to provide illuminating light to the operative site. In an exemplary configuration, endoscopic portion 26 includes an outer sheath 60 and an annular array of fiber optic elements 62 extending between light source connector 64 of endoscope housing 58 and the distal end of outer sheath 60 to illuminate the operative site. Any known light source may be connected to connector 64 to provide the illuminating light.

Endoscopic portion 26 includes an image transferring system 66 which may include CCD's, a bundle of fiber optic elements or objective lenses which transfer an optical image received at the distal end of endoscope 16 to eyepiece 68 for viewing. Alternatively, a video system including a monitor may be operatively connected to housing 58 to provide a video image of the body tissue being penetrated.

Preferably, the fiber optic elements 62 are positioned adjacent the inner wall of the outer sheath so as to surround the image transferring system. In this configuration, illumination light from the endoscope is passed through the image passing member 28 and optical images which impinge the image passing member 28 pass into the image transferring system and are relayed to eyepiece 68. An example of an endoscope which can be utilized is described in U.S. Pat. No. 4,964,710.

In an alternate embodiment, the obturator assembly 12 and endoscope 16 or optical components thereof can be a single unit inserted into cannula assembly 14. For example, the obturator assembly can be manufactured with illumination optics and/or imaging optics positioned therein so that the obturator assembly itself can function to penetrate tissue as well as to light the surgical site and transmit images to the video monitor. In this version, the obturator would not have a longitudinal bore and it would be sealed.

In operation, endoscope 16 is inserted into the trocar assembly 10, i.e. into longitudinal bore 24 of obturator sleeve 20, as shown in FIG. 4. The surgeon then positions the blade 34 against the body tissue and repeatedly moves blade 34 by continuously squeezing trigger 102 to automatically move the blade 34 rapidly from the nondeployed position to the deployed position and back to the non-deployed position. Pressure is applied to hand grip 21 in the distal direction to penetrate the body tissue. The movement of blade 34 facilitates controlled cutting of the body tissue, thus permitting the surgeon to apply relatively minimal pressure to hand grip 21 to penetrate the body tissue. At the same time the surgeon may cause the lateral translation of cutting blade 34 about the distal end of obturator 20 by manually rotating lever 160 in a first and second direction as described above. During penetration of the body tissue the surgeon either observes such penetration through eyepiece 68, or in instances where a video system is utilized the surgeon simply observes the penetration of the body tissue via any known video monitor.

Alternatively, the surgeon may also more selectively deploy the blade 34 during penetration. That is, the surgeon may insert the trocar assembly and bluntly penetrate the body tissue until reaching thicker tissue, such as muscle. At this point, the blade can be deployed and/or laterally translated to penetrate (cut through) this thick tissue. When thick tissue is again encountered, the blade can be deployed and/or laterally translated again. After penetration into the body cavity, both the endoscope 16 and the obturator assembly 12 are removed from the cannula assembly 14, leaving the cannula assembly 14 in the body for insertion of desired instrumentation therethrough.

It will be understood that various modifications can be made to the embodiments herein disclosed without departing from the scope thereof. For example, various diameters for the cannula assembly, the obturator assembly, as well as various diameter endoscopes are contemplated. Also, various modifications may be made in the configuration of the trigger assembly to achieve the instantaneous deployment and retraction of the blade. Therefore, the above description should not be construed as limiting but merely as exemplifications of preferred embodiments thereof. Those skilled in the art will envision other modifications within the scope of the claims appended hereto.

## Claims

1. An obturator (12) for penetrating tissue, which comprises :
an outer member (20) defining a longitudinal axis, and having proximal and distal ends;
a leading member (28) disposed adjacent the distal end of the outer member and having a tissue contacting outer surface; and
a blade (34) mounted adjacent the leading member,
wherein the leading member further includes an optical window adapted to permit passage of light therethrough for detection by a clinician,
**characterised in that** the blade is adapted for at least lateral traversing movement relative to the longitudinal axis to thereby traverse the outer surface of the leading member to facilitate penetrating of tissue during advancement of the leading member within the tissue.

2. The obturator according to claim 1 wherein the outer member includes a longitudinal opening adapted for reception of an endoscope.

3. The obturator according to claim 1, including an imaging device associated with the outer member, the imaging device adapted to transmit an image received through the optical window.

4. The obturator according to claim 1, wherein the blade is adapted for reciprocating movement along the outer surface of the leading member;

5. The obturator according to claim 1, wherein the outer surface of the leading member includes a channel for at least partial reception of the blade, the blade being movable within the channel.

6. The obturator according to claim 1 wherein the outer member includes an outer groove in general alignment with the channel of the leading member for at least partial reception of the blade, the blade being movable within the outer groove.

7. The obturator according to claim 6 wherein the outer member includes a pair of opposed outer grooves.

8. The obturator according to claim 1 wherein the blade is further adapted for longitudinal movement relative to the leading member to move between an initial position and an advanced position.

9. The obturator according to claim 1 including a housing connected to the proximal end of the outer member.

10. The obturator according to claim 9, including a manual actuator mounted to the housing and operatively connected to the blade, the manual actuator being dimensioned for manipulation by the clinician to cause the at least traversing movement of the blade.

11. The obturator according to claim 10, wherein the blade is adapted for longitudinal movement relative to the leading member to move between an initial position and an advanced position and further including a manual advancer mounted to the housing and operatively connected to the blade, the manual advancer being dimensioned for manipulation by the clinician to move the blade between the initial position and the advanced position.

12. The optical obturator according to claim 1, wherein the optical window defines a general hemispherically-shaped configuration.

13. The optical obturator according to claim 8, wherein the blade at least partially extends beyond the optical window in the advanced position.

## Patentansprüche

1. Obturator (12) zum Durchdringen von Gewebe, welcher umfasst:
ein äußeres Element (20), welches eine Längsachse definiert und nahe und ferne Enden aufweist;
ein führendes Element (28), welches benachbart dem fernen Ende des äußeren Elements angeordnet ist und eine Gewebe kontaktierende äußere Oberfläche aufweist; und
eine Klinge (34), welche benachbart dem führenden Element angebracht ist,
wobei das führende Element des Weiteren ein optisches Fenster umfasst, welches ausgelegt ist, um den Durchgang von Licht ebendort hindurch für das Sondieren durch einen Kliniker zuzulassen, **dadurch gekennzeichnet, dass** die Klinge wenigstens zur seitlichen Querbewegung relativ zur Längsachse ausgelegt ist, um dadurch die äußere Oberfläche des führenden Elements zu kreuzen, um das Durchdringen des Gewebes während des Vorschubs des führenden Elements im Gewebe zu ermöglichen.

2. Obturator nach Anspruch 1, wobei das äußere Element eine Längsöffnung umfasst, welche zur Aufnahme eines Endoskops ausgelegt ist.

3. Obturator nach Anspruch 1, umfassend eine bildgebende Vorrichtung, welche dem äußeren Element beigefügt ist, wobei das bildgebende Element ausgelegt ist, um ein Bild, welches durch das optische Fenster empfangen wird, zu übertragen.

4. Obturator nach Anspruch 1, wobei die Klinge für hin- und hergehende Bewegung entlang der äußeren Oberfläche des führenden Elements ausgelegt ist.

5. Obturator nach Anspruch 1, wobei die äußere Oberfläche des führenden Elements einen Kanal für wenigstens eine teilweise Aufnahme der Klinge umfasst, wobei die Klinge im Kanal bewegbar ist.

6. Obturator nach Anspruch 1, wobei das äußere Element eine äußere Nut in allgemeiner Flucht mit dem Kanal des führenden Elements für wenigstens eine teilweise Aufnahme der Klinge umfasst, wobei die Klinge in der äußeren Nut bewegbar ist.

7. Obturator nach Anspruch 6, wobei das äußere Element ein Paar von gegenüberliegenden äußeren Nuten umfasst.

8. Obturator nach Anspruch 1, wobei die Klinge des Weiteren für die Längsbewegung relativ zum führenden Element ausgelegt ist, um sich zwischen einer Ausgangsposition und einer ausgerückten Position zu bewegen.

9. Obturator nach Anspruch 1, umfassend ein Gehäuse, welches mit dem nahen Ende des äußeren Elements verbunden ist.

10. Obturator nach Anspruch 9, umfassend eine manuelle Betätigung , welche auf dem Gehäuse angebracht ist und betriebsfähig mit der Klinge verbunden ist, wobei die manuelle Betätigung zur Handhabung durch den Kliniker bemessen ist, um die wenigstens querführende Bewegung der Klinge zu verursachen.

11. Obturator nach Anspruch 10, wobei die Klinge zur Längsbewegung relativ zum führenden Element ausgelegt ist, um sich zwischen einer Ausgangsposition und einer ausgerückten Position zu bewegen, und des Weiteren einen manuellen Ausrücker umfasst, welcher am Gehäuse angebracht ist und betriebsfähig mit der Klinge verbunden ist, wobei der manuelle Ausrücker zur Handhabung durch den Kliniker bemessen ist, um die Klinge zwischen der Ausgangsposition und der ausgerückten Position zu bewegen.

12. Obturator nach Anspruch 1, wobei das optische Fenster eine allgemeine halbkugelförmige Konfiguration definiert.

13. Obturator nach Anspruch 8, wobei die Klinge sich in der ausgerückten Position wenigstens teilweise über das optische Fenster hinaus erstreckt.

## Revendications

1. Obturateur (12) pour pénétrer un tissu, qui comprend :
un élément externe (20) définissant un axe longitudinal, et possédant des extrémités proximale et distale ;
un élément de guidage (28) disposé de façon adjacente à l'extrémité distale de l'élément externe et ayant un tissu en contact avec la surface externe ; et
une lame (34) montée de manière adjacente à l'élément de guidage,
dans lequel l'élément de guidage inclut en outre une fenêtre optique conçue pour permettre le passage de lumière à travers elle pour la détection par un clinicien,
**caractérisé en ce que** la lame est conçue pour au moins un déplacement transversal latéral par rapport à l'axe longitudinal afin de traverser la surface externe de l'élément de guidage pour faciliter la pénétration du tissu lors de l'avancement de l'élément de guidage à l'intérieur du tissu.

2. Obturateur selon la revendication 1, dans lequel l'élément externe inclut une ouverture longitudinale conçue pour la réception d'un endoscope.

3. Obturateur selon la revendication 1, incluant un dispositif d'imagerie associé à l'élément externe, le dispositif d'imagerie étant conçu pour transmettre une image reçue à travers la fenêtre optique.

4. Obturateur selon la revendication 1, dans lequel la lame est conçue pour un déplacement de va-et-vient le long de la surface externe de l'élément de guidage ;

5. Obturateur selon la revendication 1, dans lequel la surface externe de l'élément de guidage inclut un canal pour au moins la réception partielle de la lame, la lame étant mobile à l'intérieur du canal.

6. Obturateur selon la revendication 1, dans lequel l'élément externe inclut une gorge externe en alignement général avec le canal de l'élément de guidage pour une réception au moins partielle de la lame, la lame étant mobile à l'intérieur de la gorge externe.

7. Obturateur selon la revendication 6, dans lequel l'élément externe inclut une paire de gorges externes opposées.

8. Obturateur selon la revendication 1, dans lequel la lame est en outre conçue pour un déplacement longitudinal par rapport à l'élément de guidage pour se déplacer entre une position initiale et une position avancée.

9. Obturateur selon la revendication 1, incluant un logement relié à l'extrémité proximale de l'élément externe.

10. Obturateur selon la revendication 9, incluant un actionneur manuel monté sur le logement et relié opérationnellement à la lame, l'actionneur manuel étant dimensionné pour la manipulation par le clinicien pour provoquer le déplacement au moins transversal de la lame.

11. Obturateur selon la revendication 10, dans lequel la lame est conçue pour un déplacement longitudinal par rapport à l'élément de guidage pour se déplacer entre une position initiale et une position avancée et incluant en outre un dispositif d'avancement manuel monté sur le logement et relié opérationnellement à la lame, le dispositif d'avancement manuel étant dimensionné pour une manipulation par le clinicien pour déplacer la lame entre la position initiale et la position avancée.

12. Obturateur optique selon la revendication 1, dans lequel la fenêtre optique définit une configuration générale de forme hémisphérique.

13. Obturateur optique selon la revendication 8, dans lequel la lame s'étend au moins partiellement au-delà de la fenêtre optique dans la position avancée.
